Europäisches Patentamt

European Patent Office

Office européen des brevets

(11) Publication number: **0 229 735**
**A2**

## (12) EUROPEAN PATENT APPLICATION

(21) Application number: **87300436.0**

(22) Date of filing: **19.01.87**

(51) Int. Cl.³: **A 61 B 5/02**
**A 61 N 1/38**

(30) Priority: **17.01.86 IE 155/86**

(43) Date of publication of application:
**22.07.87 Bulletin 87/30**

(84) Designated Contracting States:
**AT BE CH DE ES FR GB GR IT LI LU NL SE**

(71) Applicant: **Lamb, Edward**
**Cielito Blackglen Road**
**Ticknock County Dublin(IE)**

(72) Inventor: **Lamb, Edward**
**Cielito Blackglen Road**
**Ticknock County Dublin(IE)**

(74) Representative: **Freed, Arthur Woolf et al,**
**MARKS & CLERK 57-60 Lincoln's Inn Fields**
**London WC2A 3LS(GB)**

(54) Apparatus for monitoring a child's pulse rate.

(57) An apparatus for monitoring a child's pulse rate comprises means (113) for detecting the child's pulse and means (112, 116) for administering a mild electric shock to the child if the pulse rate exceeds or falls below a preset range. Preferably the shock is only administered if the period between each of a predetermined number of consecutive pulse beats falls outside preset limits corresponding to the said preset range.

EP 0 229 735 A2

Croydon Printing Company Ltd

1

## APPARATUS FOR MONITORING A
## CHILD'S PULSE RATE

This invention relates to an apparatus for monitoring a child's pulse rate, in particular an infant, in an attempt to prevent death by the so-called "cot death syndrome".

According to the invention, there is provided an apparatus for monitroing a child's pulse rate comprising means for detecting the child's pulse and means for administering a mild electric shock to the child if the pulse rate exceeds or falls below a present range.

In one embodiment of the invention, the shock is only administered if the child's pulse rate exceeds or falls below the preset range for a predetermined period of time.

However, in another embodiment, the shock is only administered if the period between each of a predetermined number of consecutive pulse beats falls outside preset limits corresponding to the said preset range.

It is not known for certain how a child's pulse rate varies prior to stopping in a cot death, but it is thought that in cot death syndrome a child's pulse rate may increase due to respiratory failure and lack of oxygen, or the pulse rate may decrease before the heart stops. Accordingly, the preset range is chosen at its widest to correspond to the theoretical safe range which is approximately 80 to 190 beats per minute, although a more restricted sub-range may be chosen.

The shock should be such as to stimulate the nervous system of the child in order to raise the consciousness level of the child, but insufficient to injure the child.

The apparatus may be of wrist-watch or bracelet form for attachment to the child's arm or leg at a position where the detecting means can receive a good signal from the child's pulse. The power supply may be a small battery so that there is no possibility, through any failure of the circuitry, of causing injury to the child by a significant electrical shock. The power supply may be switched on manually after the apparatus is affixed to the child, or automatically by a heat-sensitive switch which is brought into contact with the child when the apparatus is affixed to the child. The latter would prevent the child itself accidentally turning off the apparatus.

If the electric shock does not bring the child's pulse rate back within the preset range within a further period, a second and even third shock may be administered. After giving the second or third shock, the apparatus may be inhibited from giving any further shocks for a considerably longer period, for example 15 minutes.

The apparatus may include an indicator device, such as an LED, which is switched on and remains on when the first shock is given. This will indicate later to the parents or guardians that at least one isolated shock has been given. The apparatus may further include a remote alarm which is activated when a second shock is given closely following upon a first, to alert the parents immediately. If desired, the alarm may be activated by the first shock.

The remote alarm may be activated by a radio signal from the apparatus, thereby permitting the apparatus to be entirely devoid of leads extending from the child to a remote location. If, however, such a lead is considered allowable, the apparatus need not be of self-contained wrist-watch or bracelet construction, since all that need be affixed to the child is a means for detecting the child's pulse and a means for administering the shock(s), the rest of the apparatus being located remote from the child.

Embodiments of the invention will now be described, by way of example, with reference to the accompanying drawings, in which:

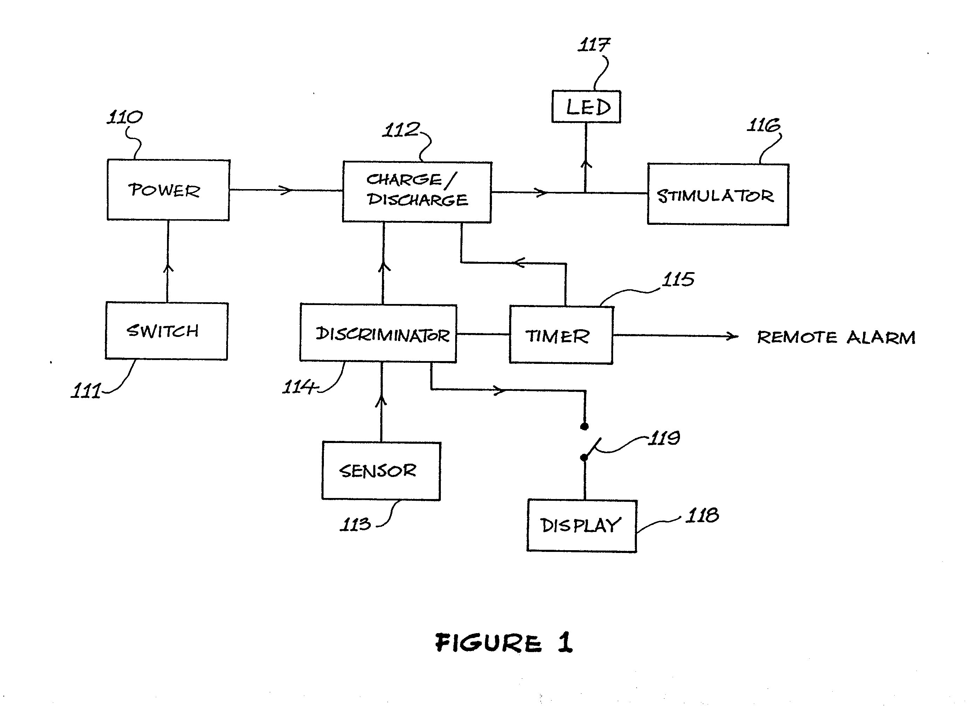

Figure 1 is a block diagram of a first embodiment of the invention,

Figure 2 is a considerably more detailed block diagram of a second embodiment of the invention, and

Figures 3 and 4 are detailed circuits of parts of figure 2.

The apparatus shown includes a power supply, such as a small battery 110, having a switch 111. The latter may be manually operated, or operated by the warmth of a child's body. The power supply is connected to a charger/discharger 112 which includes a capacitor (not shown).

A pulse sensor 113 is provided to detect the child's pulses. Signals from the sensor 113 are received by a pulse rate discriminator 114. The latter monitors the pulse rate of the child, and if the pulse rate falls below or exceeds a preset adjustable range, typically 80 - 190 beats per minute, provides a steady output signal of a given logic level to a timing (counter) circuit 115 and to the charger/discharger 112.

The timing circuit 115 is adapted to measure a 3 second period, starting upon receipt of the leading edge of the logic signal from the discriminator 114. During this period the capacitor in the charger/discharger 112 is allowed to charge up, this being enabled by the logic signal direct from the discriminator 114. At the end of the 3 second period, if the pulse rate is still outside the range set by the discriminator 114 i.e. if the output from 114 is still at the given logic level, the timing circuit 115 provides a signal to the charger/discharger 112 whereby the latter discharges through a stimulator 116 adapted to give a mild electric shock to the child.

If, however, at the end of the 3 second period the pulse rate has returned to within the preset range, as indicated by removal of the given logic level from the output of the discriminator 114, the discharge of the capacitor through the stimulator 116 is inhibited and the apparatus returns to the original state, except that the capacitor in 112 may remain charged if not discharged through a separate load.

Assuming, though, that a shock was given, the timing circuit 115 is adapted to measure a further 5 second period, during which the capacitor is again charged up. At the end of this 5 second period, the capacitor is again discharged through the stimulator 116 if the given logic level is still present at the output of the discriminator 114 i.e. if the pulse rate has not returned to within the preset range. At the same time, a remote alarm (not shown), is operated to alert the child's parents or other guardians, and the timing circuit 115 inhibits any further discharge of the capacitor for, say, 15 minutes.

However, if the pulse rate has returned to normal at the end of the 5 second period, discharge of the capacitor through the stimulator 116 is again inhibited and the apparatus returns to the original state.

Note that the alarm is raised only if the pulse rate remains outside the preset range for 8 seconds, i.e. two shocks are given after 3 and 8 seconds respectively, but not if only one shock is given in any 8 second interval. Nevertheless, in order to inform the parents that at least one isolated shock has been given, the apparatus includes an LED indicator 117 whichis switched on and remains on after a single isolated shock.Clearly it is possible to arrange for the alarm to be operated by the first shock, i.e. after 3 seconds, irrespective of any subsequent shock.

Finally, the apparatus may include a LED 118 which digitally displays the current pulse rate when a test switch 119 is closed manually.

Alternatively, the LED may continuously indicate the current pulse rate when the apparatus is switched on to provide an indication that the apparatus is operating properly. Thus, if the power supply from the battery declined the LED 118 would not function properly and the child's parents or guardians would know that a new battery was required.

It is to be understood that since the putput of the discriminator 114 is only relevant at the end of the 3 second period and at the end of the further 5 second period, it is possible for a shock to be delivered not only if the pulse rate remains above or below the preset range for the relevant period, but also if it changes from being above the range to below the range during such period.

As mentioned above, the apparatus may be manufactured using microcircuit techniques to fit on a bracelet, or it may be manufactured such that only the sensor 113 and stimulator 116 are coupled to the child and the remaining circuitry is remotely located. Further, the power supply 110 may supply the power needed to operate the other components of the apparatus, in addition to providing a charge for the capacitor in the charger/discharger 112.

Finally, it is desirable to permit adjustment not only of the preset range of the discriminator 114, but also of the periods before the first and second shocks (3 and 5 seconds respectively in the above), and of the strength of the shock itself which may be increased for the second shock.

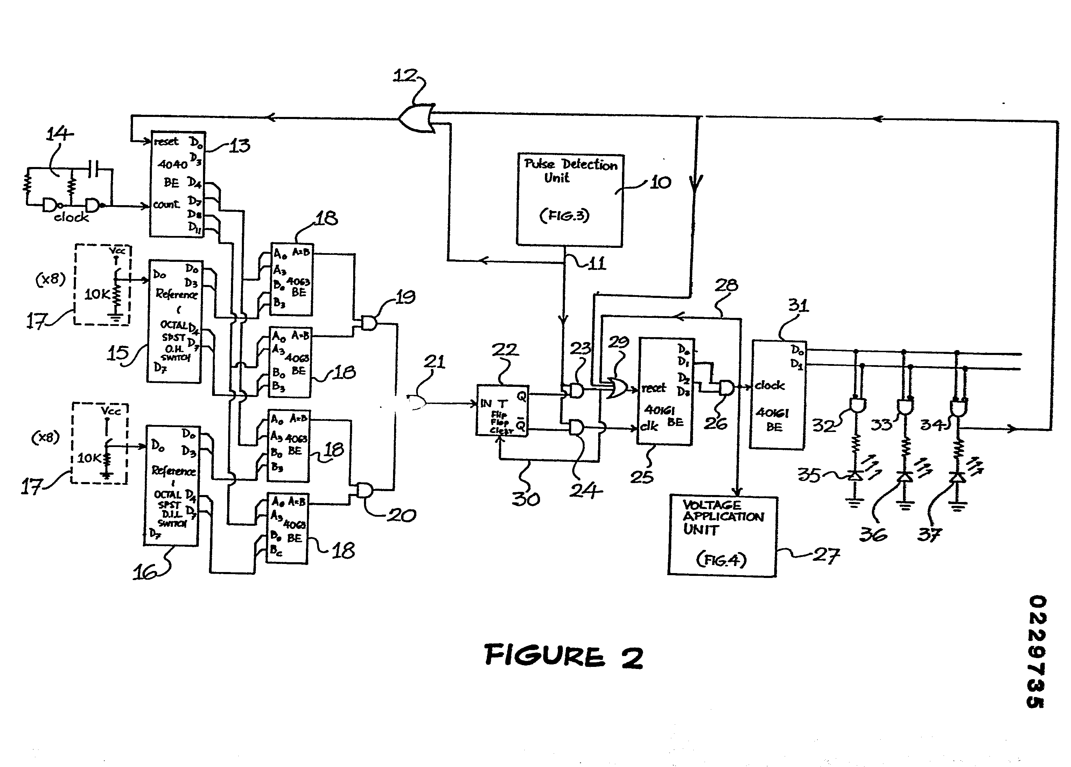

A second embodiment of the invention is shown in more detail in figures 2 to 4.

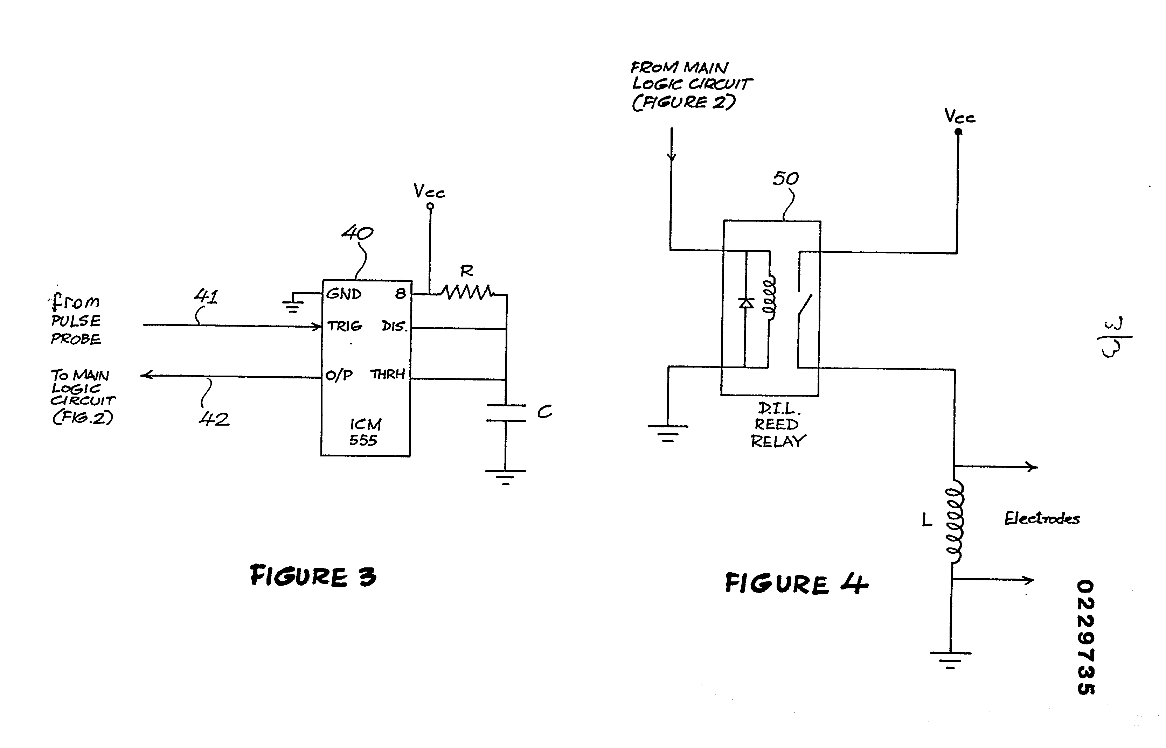

Referring to figure 2, a pulse detection unit 10, to be described more fully later with reference to figure 3, supplies at its output 11 a voltage at logic level "1" for a duration of 1 ms each time the unit 10 detects a single pulse (heartbeat). Each such 1 ms pulse signal is applied through an OR gate 12 to the reset input of a 12-bit counter 13 (type 4040 BE).

When a 1 ms pulse signal arrives at the reset input of the counter 13, the latter starts to count up from zero in response to clock signals supplied to the counting input of the counter 13 by an oscillator circuit 14. The clock signals have a frequency of about 1.6 kHz. The frequency of the oscillator circuit, and hence the frequency of the clock signals, is determined by the value of the capacitor shown in the oscillator circuit 14. Alternatively, a crystal oscillator can be used to provide the clock signals. By ignoring the least significant outputs D0 to D3 of the counter 13, the clock signals are divided by 16, so that in response to each 1 ms pulse signal the outputs D4 to D11 provide an 8-bit binary signal which counts up from 0 to a possible maximum of 255 (decimal equivalent) at a frequency of 100 Hz. Clearly, it would take just over 2.5 seconds for the counter 13 to reach its maximum value assuming that it were not reset.

Two reference bytes are provided at the outputs D0 to D7 of two octal single pole, single throw, dual-in-line (SPST DIL) switches 15 and 16 respectively. Each input D0 to D7 of each switch is connected to a respective logic "1" generator 17, only one of which is shown for each switch, and the values of the output bytes are set manually using the octal switches.

Reference byte 1, which is present at the outputs DO to D7 of the switch 15, corresponds to the count for the minimum allowable period between consecutive heartbeats. For example, if this minimum period were selected to be 1/3 second (pulse rate 180 beats per minute), the switch 15 would be set to "00100001". This corresponds to decimal 33 and is the value the counter 13 would reach about 1/3 second after resetting.

Reference byte 2, which is present at the outputs DO to D7 of the switch 16, corresponds to the count for the maximum allowable period between consecutive heartbeats. For example, if this maximum period were selected to be 2/3 second (pulse rate 90 beats per minute), the switch 16 would be set to "01000010". This corresponds to decimal 66 and is the value the counter 13 would reach about 2/3 second after resetting.

Clearly the switches 15 and 16 are effectively stores respectively establishing the minimum and maximum period allowable between consecutive heartbeats.

As the counter 13 counts up from zero to a 1 ms reset pulse signal from 10, its output is compared withthe two reference bytes. This is achieved using four 4-bit comparators 18 (type 4063 BE).

The outputs D4 to D7 of the counter 13 are connected to the inputs A0 to A3 of both the first and third comparators (counting from top to bottom), while the outputs D8 to D11 of the counter 13 are connected to the inputs A0 to A3 of the second and fourth comparators. The outputs D0 to D3 of the switch 15 are connected to the inputs B0 to B3 of the first comparator, and the outputs D4 to D7 of the switch 15 are connected to the inputs B0 to B3 of the second comparator. Similarly, the outputs D0 to D3 of the switch 16 are connected to the inputs B0 to B3 of the third comparator, and the outputs D4 to D7 of the switch 16 are connected to the inputs B0 to B3 of the fourth comparator.

Each comparator 18 is designed to give a logic "1" output when the four bits on its A inputs are identical to the four bits on its B inputs. Accordingly, with the arrangement shown, it will be seen by the inspection that each of the first and second comparators 18 will give a logic "1" output simultaneously when the count at the output of the counter 13 reaches the value of the reference byte 1 stored by the switch 15, and each of the third and fourth comparators 18 will give a logic "1" output simultaneously when the count at the output of the counter 13 reaches the value of the reference byte 2 stored by the switch 16.

The outputs of the first and second comparators are connected to a first AND gate 19, and the outputs of the third and fourth comparators are connected to a second AND gate 20. Accordingly, when the count from 13 reaches the value of the reference byte 1 this will be indicated by a logic "1" at the output of the AND gate 19, whereas when the count from 13 reaches the value of the reference byte 2 this will be indicated by a logic "1" at the output of the AND gate 20.

The outputs from the AND gates 19 and 20 are supplied through an OR gate 21 to a T flip-flop 22. The output O of the T flip'flop 22 is always at or set to zero at the start of counting by the counter 13, as will be described.

Accordingly, upon the application of a 1 ms reset pulse signal from 10 to the counter 13, and ignoring for the moment the effect of a subsequent reset pulse signal from 10, the output $\bar{Q}$ of the flip-flop 22 will go from "0" to "1" when the count from 13 reaches the value of the reference byte 1, and go back from "1" to "0" when the count reaches the value of the reference byte 2. The inverse output $\bar{O}$ of the flip-flop will at the same time go from "1" to "0" and back to "1".

Therefore, the criterion for determining that the child's pulse rate is within acceptable limits, as set by the two reference bytes, is that the output Q of the flip-flop 22 should be at logic "1" when the next 1 ms pulse signal is provided by the unit 10.

Each 1 ms pulse signal from 10 is supplied not only to the counter 13 but also to one input of each of two AND gates 23 and 24, the other input of the AND gate 23 being connected to the Q output of the flip-flop 22 and the other input of the and gate 24 being connected to the Q output of the flip-flop 22.

Accordingly, when a particular 1 ms reset pulse signal has set the counter 13 counting, the next such pulse signal will not only reset the counter 13 once again, but also enable the and gates 23 and 24. Therefore, if the Q output of the flip-flop 22 is at logic "1" at this time, indicating that the child's heartbeat rate is outside the preset limits (i.e. below reference byte 1 or above reference byte 2), the AND gate 24 will supply a logic "1" to the clock input of a 4-bit counter 25 (type 40161 BE).

Assuming that the child's pulse rate remains outside the preset limits, the above sequence of events will recur and the counter 25 will count the number of consecutive pulses for which the child's pulse rate remains outside the preset limits, as indicated by the number of consecutive logic "1's" at the output of the AND gate 24.

When six such consecutive pulses have been detected, the counter 25 will provide a logic "1" on both its outputs D1 and D2, thereby activating a voltage application unit 27 _via_ an AND gate 26 for administering a mild shock to the child. At the same time the counter is reset _via_ a feedback line 28 and an OR gate 29 ready for counting a further set of six consecutive pulses for which the child's pulse rate remains outside the preset limits.

However, if at any time before six consecutive pulses have been counted by the counter 25, any one or more pulse signals from 10 follows the preceding pulse signal by a period which is within the preset limits defined by the two reference bytes, the AND gate 23 will give a logic "1" rather than the AND gate 24, since the output Q of the flip-flop 22 will be at logic "1" and the output $\overline{Q}$ at logic "0". The signal from the AND gate 23 will therefore reset the counter 25 via the OR gate 29, and also reset the output Q of the flip-flop 22 to "0" via the feedback line 30.

The net result is therefore that while the period between pulses remains within the limits set by the reference bytes, the output Q of the flip-flop 22 will always be at logic "1" and the output $\overline{Q}$ at logic "0" when the pulse signals are supplied by the unit 10 to the AND gates 23 and 24, so that no counting occurs by the counter 25. However, any single pulse signal from 10 which follows the previous pulse signal by a period outside the preset limits will find the logic levels at the outputs Q and $\overline{Q}$ of the flip-flop 22 reversed, so that the AND gate 24 supplies a signal to the counter 25 for counting.

Nevertheless, this will not in itself cause a shock to be administered to the child since it is necessary for the counter 25 to count six such signals consecutively from the AND gate 24 before activating the unit 27. As described above, if less than six such signals have been counted, any one pulse signal received from the unit 10 which is within the preset period will reset the counter 25 back to zero.

A second counter 31 (type 40161 BE) counts the number of shocks administered, and a set of AND gates 31 to 34 decodes the number of shocks given. A first light emitting diode (LED) 35 is switched on by the AND gate 32 when the first shock is given, a second LED 36 is switched on by the AND gate 33 when the second shock is given, and a third LED 37 is switched on by the AND gate 34 when the third shock is given. The signal provided by the AND gate 34 upon giving the third shock also holds the counter 13 reset <u>via</u> the OR gate 12, thereby limiting the number of shocks given to three.

The signal from the same AND gate 34, or indeed from any of the three AND gates 32 to 34, may also activate a remote alarm to alert the child's parents or other guardians.

Referring to figure 3, the pulse detection unit 10 comprises a timer 40 (type ICM 555 in monostable mode). The timer 40 is responsive to each pulse signal on line 41 from a probe (not shown) attached at a convenient location to the child's body to provide a short pulse signal to the main logic circuit of figure 2 <u>via</u> an output line 42. As mentioned before, the duration of the pulse signal on the line 42 is 1 ms, which is obtained by selecting the value of the resistor $R = 1\ m$ and the value of the capacitor $C = 1\ nF$. The value of the voltage supply $V_{cc}$ is between 3 to 18 volts.

The probe which is affixed to the child's body may be a commercially available miniature IR plethysograph.

Figure 4 is a circuit diagram of the voltage application unit 27 of figure 2.

The signal from the AND gate 26 (figure 2) closes a dual-in-line reed relay 50. The rapid increase in current through the inductor L causes a voltage pulse $V = L.dI/dt$ to appear across the inductor, which is applied to the child across a pair of electrodes in the form of small conductive pads (not shown). The value of the inductor L in relation to $V_{cc}$ is such that a shock is given of a level sufficient so as to stimulate the nervous system of the child in order to raise the consciousness level of the child, but insufficient to injure the child.

It is to be understood that although the circuit show in figures 2 to 4 has been described in terms of commerically obtainable components, apart from the pulse probe, electrodes and inductor L, all else may be provided on a ULA or custom chip, or as surface mounted discrete components. Thus, it is technically possible to build the entire circuit in a bracelet for attachment to a child's arm or leg.

CLAIMS:-

1. An apparatus for monitoring a child's pulse rate, charterised by means for detecting the child's pulse and means for administering a mild electric shock to the child if the pulse rate exceeds or falls below a preset range.

2. An apparatus according to claim 1, wherein the shock is only administered if the child's pulse rate exceeds or falls below the preset range for a predetermined period of time.

3. An apparatus according to claim 1, wherein the shock is only administered if the period between each of a predetermined number of consecutive pulse beats falls outside preset limits corresponding to the said preset range.

4. An apparatus according to claim 3, wherein the apparatus comprises a source (14) of clock signals, a counter (13) for counting the clock signals, the counter being reset to a predetermined value upon each detection of the child's pulse beat, means (15, 16) for storing first and second count values corresponding respectively to the minimum and maximum permissible period between consecutive pulse beats, means (18 to 20) for comparing the current counter value with each of the first and second stored values, means (21 to 24) responsive to the comparison means for generating a first signal if the counter value is less than the first stored value or greater than the second stored value when a pulse beat is detected, and a further counter (25) for counting the number of first signals, the further counter generating a second signal to enable a shock to be administered when the said predetermined number of first signals have been generated in respect of consecutive pulse beats.

**0229735**

5. An apparatus according to claim 4, wherein the said second signal resets the further counter (25).

6. An apparatus according to claim 4, wherein the means (21 to 24) responsive to the comparison means (18 to 20) is adapted to reset the further counter (25) if a first signal is not generated when a pulse beat is detected.

7. An apparatus according to claim 4, wherein the comparison means comprises means (18,19) for generating a third signal when the counter value reaches the first stored value and means (18,20) for generating a fourth signal when the counter value reaches the second stored value, and wherein the means responsive to the comparison means comprises a bistable device (22) which changes from a first state to a second state in response to the third signal and from the second state back to the first state in response to the fourth signal, and gating means (23,24) connected to the bistable device (22) which generates a said first signal when the bistable device is in the first state and a pulse beat is detected.

8. An apparatus according to claim 7, wherein the gating means (23, 24) is adapted to reset the further counter (25) when the bistable device (22) is in the second state and a pulse beat is detected.

9. An apparatus according to claim 4, further including a third counter (31) adapted to count the number of second signals, and to provide a fifth signal inhibiting further administration of shocks after a predetermined number of the second signals have been counted.

10. An apparatus according to claim 9, wherein the said fifth signal prevents counting by the first counter (13).

11. An apparatus according to claim 9, further including at least one indicator (35 to 37) associated with the third counter (31) to indicate that at least one shock has been administered.

**FIGURE 1**

FIGURE 2

FIGURE 3

FIGURE 4

3/3

0229735